# DEMANDE DE BREVET EUROPEEN

(11) **EP 4 785 851 A1**
(43) Date de publication de la demande: **05.08.2026**
(21) Numéro de dépôt: 26150225.6
(22) Date de dépôt: 05.01.2026
(51) Int. Cl.: A61B 3/028

(54) **APPAREIL DE CONTRÔLE DE LA VISION**

(30) Priorité: 30.01.2025 FR 2500976
(71) Demandeur: FIM Medical, 69100 Villeurbanne (FR)
(72) Inventeur: ROZET, Gilbert, 69780 Saint-Pierre-de-Chandieu (FR)
(74) Mandataire: Germain Maureau

(57) **Abrégé**

Appareil de contrôle de la vision d'un patient (10, 100, 200) prévu pour pouvoir occuper une première configuration dans laquelle un premier chemin optique (30, 130, 230) est ménagé entre une position d'observation d'un œil (50, 150, 250) du patient et un dispositif d'affichage d'image (70, 170, 270) et comprenant un organe mobile (13, 13', 113, 213) présentant au moins deux logements d'organe mobile (13.2, 13.2', 113.2, 213.2) destinés à occuper alternativement sur le premier chemin optique (30, 130, 230) une position de logement par rotation de l'organe mobile (13, 13', 113, 213) autour d'un axe de rotation d'organe mobile (13.1, 113.1, 213.1) sensiblement coplanaire et parallèle à une direction d'une portion du premier chemin optique (30, 130, 230) s'étendant entre la position d'observation de l'œil (50, 150, 250) du patient et la position de logement.

## Description

### Domaine technique

L'invention, qui appartient au domaine des appareils d'ophtalmologie, concerne en particulier un appareil de test de la vision permettant de dépister d'éventuels défauts visuels que présente un patient et/ou d'effectuer un diagnostic concernant la vision du patient.

### Préambule

Un dépistage et un diagnostic exhaustifs des défauts visuels d'un patient impliquent nécessairement la réalisation de différents tests en vision de loin, de près ainsi qu'en vision intermédiaire.

Que ce soit en vision de près ou de loin, les législations nationales de différents pays imposent généralement de réaliser des tests en simulant différentes distances.

Concernant par exemple les tests réalisés en médecine du travail, la distance à simuler pour tester la vision de près peut aller, selon les pays, de 25 à 40 centimètres, en passant par 33 centimètres.

Or, les appareils existants permettant de tester à la fois les visions proche et lointaine n'offrent qu'un nombre limité de distances pouvant être simulées, ce qui est un frein à leur commercialisation à l'international.

Dans certains contextes, il est par ailleurs parfois nécessaire de recourir à plusieurs appareils pour tester de manière complète un seul et même patient, ce qui est particulièrement contraignant et implique un surcoût pour le praticien.

Il existe donc un besoin de fournir un appareil de contrôle de la vision plus modulable, c'est-à-dire pouvant notamment simuler davantages de distances.

Les appareils de contrôle de la vision connus sont par ailleurs généralement volumineux, ce qui rend difficiles tant leur transport que leur stockage, et il est en outre courant que le patient lui-même doive réaliser des manipulations sur l'appareil alors même qu'il se trouve en situation de dépistage.

Il existe donc un besoin de fournir un appareil de contrôle de la vision à la fois plus compact et simple d'utilisation, c'est-à-dire ne nécessitant pas de manipulations particulières de la part du patient testé.

### Résumé de l'invention

La présente invention vise à remédier à tout ou partie des inconvénients précités.

A cet effet, la présente invention offre un appareil de contrôle de la vision d'un patient à la fois plus compact, simple d'utilisation et modulable que les appareils existants, et qui permet de réaliser de manière exhaustive des opérations de dépistage de défauts visuels que présente le patient et/ou des opérations de diagnostic précis de la vision du patient.

Selon un premier aspect, la présente invention a pour objet un appareil de contrôle de la vision d'un patient apte à occuper au moins une première configuration dans laquelle un premier chemin optique est ménagé entre une position d'observation d'un œil du patient et un dispositif d'affichage d'image, l'appareil de contrôle comprenant un organe mobile présentant au moins deux logements d'organe mobile destinés à occuper alternativement sur le premier chemin optique une position de logement par rotation de l'organe mobile autour d'un axe de rotation d'organe mobile sensiblement coplanaire et parallèle à une direction d'une portion du premier chemin optique s'étendant entre la position d'observation de l'œil du patient et la position de logement.

Selon un mode de réalisation, l'appareil de contrôle comprend en outre un élément optique primaire de premier chemin optique disposé sur le premier chemin optique.

Selon une possibilité, l'élément optique primaire de premier chemin optique est disposé entre la position d'observation de l'œil du patient et la position de logement.

Selon une autre possibilité, l'élément optique primaire de premier chemin optique est disposé entre la position de logement et le dispositif d'affichage d'image. Selon cette possibilité, la position de logement est située entre la position d'observation de l'œil du patient et l'élément optique primaire de premier chemin optique.

Selon un mode de réalisation, chacun desdits au moins deux logements d'organe mobile intègre en permanence un élément optique secondaire de premier chemin optique, ou bien chacun desdits au moins deux logements d'organe mobile est apte à accueillir temporairement un élément optique secondaire de premier chemin optique.

Selon une possibilité, l'appareil de contrôle peut comprendre, sur le premier chemin optique, un élément optique secondaire de premier chemin optique et être dépourvu d'élément optique primaire de premier chemin optique.

Selon une autre possibilité, l'appareil de contrôle peut comprendre, sur le premier chemin optique, un élément optique primaire de premier chemin optique ainsi qu'un élément optique secondaire de premier chemin optique.

Selon un mode de réalisation, l'élément optique primaire de premier chemin optique est une lentille primaire de premier chemin optique.

Selon un mode de réalisation, l'élément optique secondaire de premier chemin optique est une lentille secondaire de premier chemin optique ou un prisme de premier chemin optique.

Selon un mode de réalisation, l'appareil de contrôle est apte à occuper en outre une deuxième configuration dans laquelle un deuxième chemin optique est ménagé entre la position d'observation de l'œil du patient et le dispositif d'affichage d'image, l'appareil de contrôle comprenant en outre un système de renvoi d'image comprenant un premier miroir et un deuxième miroir.

Selon un mode de réalisation, le système de renvoi d'image est au moins partiellement mobile pour pouvoir faire passer l'appareil de contrôle de la première configuration à la deuxième configuration et vice-versa.

Selon un mode de réalisation, le deuxième miroir est mobile entre une position déployée de deuxième miroir occupée par le deuxième miroir lorsque l'appareil de contrôle occupe la première configuration, et une position escamotée de deuxième miroir occupée par le deuxième miroir lorsque l'appareil de contrôle occupe la deuxième configuration.

Selon un mode de réalisation, l'appareil de contrôle comprend au moins un élément optique de deuxième chemin optique disposé sur le deuxième chemin optique.

Selon un mode de réalisation, l'au moins un élément optique de deuxième chemin optique comprend un prisme de deuxième chemin optique et/ou une lentille de deuxième chemin optique.

Selon un mode de réalisation, le système de renvoi d'image comprend un troisième miroir.

Selon un mode de réalisation, lorsque l'appareil de contrôle occupe la première configuration, un premier chemin optique supplémentaire est ménagé entre une position d'observation de l'autre œil du patient et le dispositif d'affichage d'image, l'appareil de contrôle comprend un organe mobile supplémentaire comprenant au moins deux logements d'organe mobile supplémentaire destinés à occuper alternativement sur le premier chemin optique supplémentaire une position de logement supplémentaire, par rotation de l'organe mobile supplémentaire autour d'un axe de rotation d'organe mobile supplémentaire sensiblement coplanaire et parallèle à une direction d'une portion du premier chemin optique supplémentaire s'étendant entre la position d'observation de l'autre œil du patient et la position de logement supplémentaire.

Selon une possibilité, le premier chemin optique correspond à un chemin optique de vision de loin et ladite première configuration correspond à une configuration de vision de loin, tandis que le deuxième chemin optique correspond à un chemin optique de vision de près et ladite deuxième configuration correspond à une configuration de vision de près.

Selon cette dernière possibilité, l'appareil de contrôle présente l'avantage de permettre à un patient, depuis une seule position d'observation, de pouvoir observer des images affichées sur un dispositif d'affichage d'image via un chemin optique de vision de loin et via un chemin optique de vision de près, et ce sans risque de confusion pour le patient.

En effet, le système de renvoi d'image est apte à renvoyer les images affichées sur le dispositif d'affichage d'image vers la position d'observation de l'œil du patient, et les images renvoyées atteignent l'œil du patient en empruntant le chemin optique de vision de loin (ou premier chemin optique) lorsque l'appareil de contrôle occupe la configuration de vision de loin ou première configuration, et en empruntant le chemin optique de vision de près (ou deuxième chemin optique) lorsque l'appareil de contrôle occupe la configuration de vision de près ou deuxième configuration.

Selon une possibilité, l'organe mobile présente au moins cinq logements d'organe mobile, et lesdits au moins cinq logements d'organe mobile sont aptes à accueillir chacun un élément optique, comme par exemple une lentille.

C'est en particulier la configuration de l'organe mobile, présentant un axe de rotation coplanaire et parallèle à la portion du premier chemin optique s'étendant entre la position d'observation de l'œil du patient et la position de logement, qui permet d'agencer autant de logements d'organe mobile, tout en offrant une compacité accrue de l'appareil de contrôle.

Grâce à ces dispositions, l'appareil de contrôle est capable de simuler, en particulier dans la première configuration, davantage de distances de test que les appareils existants.

Ainsi, l'appareil de contrôle est plus modulable que les appareils de connus, sans pour autant que sa structure soit plus encombrante ni sa fabrication plus complexe.

De par sa mobilité en rotation autour de l'axe de rotation d'organe mobile, l'organe mobile est apte à faire occuper, tour à tour, la position de logement à chacun des cinq logements d'organe mobile.

Selon une possibilité, l'appareil de contrôle comprend des moyens de définition de la position d'observation de l'œil du patient.

Selon cette possibilité, les moyens de définition de la position d'observation de l'œil du patient comprennent un système de support, de positionnement et/ou de maintien en position de la tête du patient, comme par exemple une têtière ou une mentonnière, ou encore des lunettes de visée.

Selon une possibilité, l'appareil de contrôle comprend un dispositif d'affichage d'image permettant par exemple d'afficher des optotypes ou autres symboles observables par l'œil du patient depuis la position d'observation de l'œil du patient.

Selon cette possibilité, le dispositif d'affichage d'image est intégré à l'appareil de contrôle.

Selon une autre possibilité, l'appareil de contrôle est prévu pour fonctionner avec un dispositif d'affichage d'image déporté.

Selon cette possibilité, l'appareil de contrôle n'intègre pas de dispositif d'affichage d'image.

Selon une possibilité, l'appareil de contrôle est prévu pour fonctionner avec un dispositif d'affichage d'image supplémentaire, en plus du dispositif d'affichage d'image.

Selon cette possibilité, le dispositif d'affichage d'image supplémentaire peut être identique au dispositif d'affichage d'image.

Selon une possibilité, la lentille primaire de premier chemin optique est montée sur un support de lentille primaire de premier chemin optique, ledit support étant fixe.

En particulier, la lentille primaire de premier chemin optique est destinée à coopérer alternativement avec chacun desdits au moins cinq logements d'organe mobile accueillant (ou non) chacun par exemple une lentille secondaire de premier chemin optique distincte, de manière à former différents agencements optiques.

Selon une possibilité, l'organe mobile prend la forme d'un barillet circulaire, et en particulier celle d'un disque présentant une faible épaisseur.

Selon une possibilité, l'organe mobile présente cinq logements d'organe mobile, pour accueillir des éléments optiques, répartis régulièrement sur la surface de l'organe mobile, chacun des cinq logements d'organe mobile étant donc apte à accueillir une lentille secondaire de premier chemin optique.

Selon une autre possibilité, l'organe mobile présente six logements d'organe mobile, pour accueillir des éléments optiques, répartis régulièrement sur la surface de l'organe mobile.

Selon une possibilité, l'appareil de contrôle comprend un boîtier destiné à abriter et à protéger au moins certains des éléments de l'appareil de contrôle, le dispositif d'affichage d'image pouvant par exemple être prévu sur l'une des faces du boîtier, la position d'observation de l'œil du patient étant située à l'extérieur dudit boîtier.

Selon une possibilité, au moins deux ouvertures sont prévues dans le boîtier, respectivement pour ménager le premier chemin optique et pour ménager le deuxième chemin optique.

En particulier, au moins une ouverture de premier chemin optique est prévue pour ménager le premier chemin optique, et au moins une ouverture de deuxième chemin optique est prévue pour ménager le deuxième chemin optique.

Selon une possibilité, la lentille primaire de premier chemin optique est disposée sur le premier chemin optique entre la position d'observation de l'œil du patient et la position de logement.

Dans le mode de réalisation dans lequel chacun desdits au moins cinq logements d'organe mobile intègre en permanence une lentille secondaire de premier chemin optique, une lentille secondaire de premier chemin optique est fixée de manière permanente au sein de chacun des au moins cinq logements d'organe mobile, et il n'est pas possible de retirer ou de la remplacer.

Dans ce mode de réalisation, l'organe mobile peut avantageusement être réalisé en monobloc avec lentilles intégrées, par exemple par injection thermoplastique, ce qui est pertinent en termes de coût de fabrication.

Alternativement, chacune des lentilles secondaires de premier chemin optique peut respectivement être collée dans chacun des au moins cinq logements d'organe mobile.

Le mode de réalisation dans lequel chacun desdits au moins cinq logements d'organe mobile est apte à accueillir temporairement une lentille secondaire de premier chemin optique est avantageux en ce qu'il permet d'inter changer ou de remplacer les lentilles secondaires de premier chemin optique, pour encore plus de modularité de l'appareil de contrôle, et aussi parce qu'il permet un agencement optique dans lequel la lentille primaire de premier chemin optique n'est associée à aucune lentille secondaire de premier chemin optique.

Selon ce mode de réalisation, l'organe mobile présente, au niveau de chacun des logements, un système de fixation temporaire pour élément optique.

Dans ce mode de réalisation, chaque lentille peut par exemple être clippée, clipsée ou encore vissée dans un logement d'organe mobile, respectivement.

Selon une possibilité, le système de renvoi d'image est tel que le deuxième miroir est mobile, et par exemple mobile en rotation.

Selon une possibilité, le premier miroir est fixe, et en particulier monté sur un support de premier miroir fixe.

Selon une possibilité, le deuxième miroir est monté sur un support de deuxième miroir mobile en rotation autour d'un axe de rotation de support de deuxième miroir, le support de deuxième miroir étant apte à faire passer le deuxième miroir de la position déployée de deuxième miroir à la position escamotée de deuxième miroir et vice versa.

Selon une possibilité, le support de deuxième miroir est motorisé, et l'appareil de contrôle comprend un dispositif d'actionnement de support de deuxième miroir et un dispositif d'entrainement de support de deuxième miroir comprenant au moins un moteur.

Selon une possibilité, lorsqu'il occupe la position déployée de deuxième miroir, le deuxième miroir obstrue le deuxième chemin optique.

Selon une possibilité, lorsqu'il occupe la position escamotée de deuxième miroir, le deuxième miroir libère le deuxième chemin optique, tandis que le premier chemin optique est interrompu.

Selon une possibilité, le prisme de deuxième chemin optique est disposé sur le deuxième chemin optique entre le dispositif d'affichage d'image et la position de l'œil du patient.

Selon une possibilité, le prisme de deuxième chemin optique, monté de manière amovible sur un support de prisme de deuxième chemin optique, est interchangeable. Selon cette possibilité, différents prismes de deuxième chemin optique peuvent être utilisés avec l'appareil de contrôle.

Selon une possibilité avantageuse, l'appareil de contrôle comprend à la fois un prisme de deuxième chemin optique et une lentille de deuxième chemin optique disposés sur le deuxième chemin optique, ce qui permet un agencement optique particulier.

Selon une possibilité, la lentille de deuxième chemin optique est disposée sur le deuxième chemin optique entre le dispositif d'affichage d'image et le prisme de deuxième chemin optique.

Selon une possibilité, la lentille de deuxième chemin optique, montée de manière amovible sur un support de lentille de deuxième chemin optique, est interchangeable.

Cette dernière possibilité est avantageuse en ce qu'elle permet différents agencements optiques, c'est-à-dire différentes association prisme - lentille, sans avoir à changer de prisme de deuxième chemin optique.

Selon une possibilité, le troisième miroir est disposé sur le premier chemin optique entre le dispositif d'affichage d'image et le deuxième miroir lorsque le deuxième miroir occupe la position déployée de deuxième miroir, c'est-à-dire lorsque l'appareil de contrôle occupe la première configuration.

En outre, lorsque le deuxième miroir occupe la position escamotée de deuxième miroir, c'est-à-dire lorsque l'appareil de contrôle occupe la deuxième configuration:
- Le troisième miroir est disposé sur le deuxième chemin optique entre le dispositif d'affichage d'image et le prisme de deuxième chemin optique lorsque l'appareil de contrôle ne comprend pas de lentille de deuxième chemin optique;
- Le troisième miroir est disposé sur le deuxième chemin optique entre le dispositif d'affichage d'image et la lentille de deuxième chemin optique lorsque l'appareil de contrôle comprend une lentille de deuxième chemin optique.

Selon une possibilité, le troisième miroir est fixe, c'est-à-dire par exemple monté sur un support de troisième miroir, ledit support étant fixe.

Selon certains modes de réalisation exposés ci-dessus, l'appareil de contrôle présente l'avantage de pouvoir tester globalement la vision du patient, et donc par exemple de dépister simultanément d'éventuels défauts visuels impactant l'un ou l'autre des yeux du patient.

Ainsi et selon une possibilité, l'appareil de contrôle comprend une lentille primaire de premier chemin optique supplémentaire disposée sur le premier chemin optique supplémentaire, et par exemple située entre la position d'observation de l'autre œil du patient et la position de logement supplémentaire.

Selon une possibilité, la lentille primaire de premier chemin optique supplémentaire est identique à la lentille primaire de premier chemin optique.

Selon une possibilité, lorsque l'appareil de contrôle occupe la deuxième configuration, un deuxième chemin optique supplémentaire est ménagé entre la position d'observation de l'autre œil du patient et le dispositif d'affichage d'image.

Le système de renvoi d'image est également apte à renvoyer les images affichées sur le dispositif d'affichage d'image vers la position d'observation de l'autre œil du patient, et les images renvoyées atteignent l'autre œil du patient en empruntant le premier chemin optique supplémentaire lorsque l'appareil de contrôle occupe la première configuration, et en empruntant le deuxième chemin optique supplémentaire lorsque l'appareil de contrôle occupe la deuxième configuration.

Selon une possibilité, l'appareil de contrôle comprend un prisme de deuxième chemin optique supplémentaire disposé sur le deuxième chemin optique supplémentaire.

Selon une possibilité, le prisme de deuxième chemin optique supplémentaire est identique au prisme de deuxième chemin optique.

Selon une possibilité, le prisme de deuxième chemin optique supplémentaire est disposé sur le deuxième chemin optique supplémentaire entre le dispositif d'affichage d'image et la position de l'autre oeil du patient.

Selon une possibilité, l'appareil de contrôle comprend une lentille de deuxième chemin optique supplémentaire disposée sur le deuxième chemin optique supplémentaire.

Selon encore une possibilité, l'appareil de contrôle comprend à la fois un prisme de deuxième chemin optique supplémentaire et une lentille de deuxième chemin optique supplémentaire disposés sur le deuxième chemin optique supplémentaire.

Selon cette possibilité, la lentille de deuxième chemin optique supplémentaire est par exemple disposée sur le deuxième chemin optique supplémentaire entre le dispositif d'affichage d'image et le prisme de deuxième chemin optique supplémentaire.

Selon un mode de réalisation, l'organe mobile et l'organe mobile supplémentaire sont configurés entre eux pour être mobiles en rotation de manière synchronisée.

Selon ce mode de réalisation, un mouvement en rotation de l'organe mobile (respectivement de l'organe mobile supplémentaire) entraine en rotation, de manière synchronisée, l'organe mobile supplémentaire (respectivement l'organe mobile). Ainsi, lorsque l'organe mobile se déplace en rotation à une vitesse donnée, l'organe mobile se déplace également en rotation à la même vitesse, et en sens inverse.

Ce mode de réalisation est avantageux en ce qu'il rend superflues d'éventuelles manipulations supplémentaires de l'appareil de contrôle par un opérateur ou le patient lui-même.

La mobilité en rotation synchronisée de l'organe mobile et de l'organe mobile supplémentaire est obtenue grâce à un mécanisme d'engrenage, une première partie de mécanisme d'engrenage étant prévue sur l'organe mobile, et une deuxième partie de mécanisme d'engrenage, complémentaire de la première partie de mécanisme d'engrenage, étant prévue sur l'organe mobile supplémentaire.

Par exemple, la première partie de mécanisme d'engrenage comprend un premier système de dents par exemple disposé à la périphérie de l'organe mobile, et la deuxième partie de mécanisme d'engrenage comprend un deuxième système de dents complémentaire dudit premier système de dents et par exemple disposé à la périphérie de l'organe mobile supplémentaire.

L'appareil de contrôle peut comprendre en outre des moyens d'actionnement et des moyens d'entrainement en rotation de l'organe mobile et/ou de l'organe mobile supplémentaire.

Selon un mode de réalisation, l'appareil de contrôle comprend un mécanisme d'entrainement apte à entrainer simultanément en rotation l'organe mobile et l'organe mobile supplémentaire, ledit mécanisme d'entrainement comprenant par exemple un moteur.

Selon ce mode de réalisation, la mobilité en rotation synchronisée de l'organe mobile et de l'organe mobile supplémentaire est obtenue grâce au mécanisme d'entrainement, et lorsque l'organe mobile se déplace en rotation à une vitesse donnée, l'organe mobile se déplace également en rotation à la même vitesse.

Selon un mode de réalisation, le mécanisme d'entrainement comprend une première partie de mécanisme d'entrainement prévue sur l'organe mobile, une deuxième partie de mécanisme d'entrainement prévue sur l'organe mobile supplémentaire, et une troisième partie de mécanisme d'entrainement comprenant un pignon mobile en rotation.

Selon ce mode de réalisation, la troisième partie de mécanisme d'entrainement est complémentaire à la fois de la première partie de mécanisme d'entrainement et de la deuxième partie de mécanisme d'entrainement.

Par exemple, la première partie de mécanisme d'entrainement comprend un premier système de dents disposé à la périphérie de l'organe mobile, la deuxième partie de mécanisme d'entrainement comprend un deuxième système de dents disposé à la périphérie de l'organe mobile supplémentaire, et le pignon comprend un troisième système de dents complémentaire à la fois du premier système de dents et du deuxième système de dents.

Grâce à cet agencement et en particulier au pignon mobile en rotation, le sens de rotation de l'organe mobile supplémentaire est identique au sens de rotation de l'organe mobile. De plus, l'organe mobile supplémentaire peut par exemple être strictement identique à l'organe mobile.

Selon une possibilité, le pignon présente une forme de disque de faible épaisseur et comprend à sa périphérie le troisième système de dents.

Selon une possibilité, le pignon est motorisé, et l'appareil de contrôle comprend donc un dispositif d'actionnement de pignon et un dispositif d'entrainement de pignon comprenant au moins un moteur, ce qui réduit avantageusement encore davantage les manipulations à effectuer par l'utilisateur ou un opérateur sur l'appareil de contrôle.

Selon une autre possibilité, l'organe mobile et l'organe mobile supplémentaire sont configurés pour être mobiles en rotation indépendamment l'un de l'autre, et leurs mouvements respectifs sont désynchronisés.

Selon un deuxième aspect, la présente invention a pour objet un procédé d'utilisation de l'appareil de contrôle de la vision d'un patient précédemment décrit, ladite utilisation ayant pour objectif de dépister au moins un défaut visuel impactant l'un et/ou l'autre des yeux du patient, et/ou de réaliser un diagnostic concernant la vision du patient.

Le procédé d'utilisation de l'appareil de contrôle peut comprendre les étapes suivantes :
Un patient se place en situation d'utilisation de l'appareil de contrôle, de manière à ce qu'un œil du patient soit disposé à la position d'observation de l'œil du patient, et de manière à ce que l'autre œil du patient soit disposé à la position d'observation de l'autre œil du patient.

L'appareil de contrôle est amené à occuper la deuxième configuration.

Le dispositif d'affichage d'image affiche une première image, et le patient tente de déchiffrer ladite première image affichée par le dispositif d'affichage d'image et renvoyée par le système de renvoi d'image de l'appareil de contrôle via le deuxième chemin optique.

L'appareil de contrôle est amené à occuper la première configuration.

Le dispositif d'affichage d'image affiche une deuxième image, et le patient tente de déchiffrer ladite deuxième image affichée par le dispositif d'affichage d'image et renvoyée par le système de renvoi d'image de l'appareil de contrôle via le premier chemin optique.

L'organe mobile et l'organe mobile supplémentaire sont entrainés en rotation, et le dispositif d'affichage d'image affiche une troisième image, et le patient tente de déchiffrer ladite troisième image affichée par le dispositif d'affichage d'image et renvoyée par le système de renvoi d'image de l'appareil de contrôle via le premier chemin optique.

Les différents aspects définis ci-dessus non incompatibles peuvent être combinés.

### Brève description des figures

L'invention sera mieux comprise à la lecture de la description détaillée, laquelle est effectuée ci-dessous en rapport avec les figures, parmi lesquelles :
[Fig.1] représente, selon une vue en perspective, un appareil de contrôle de la vision d'un patient selon un premier exemple de réalisation de l'invention.
[Fig.2] représente, selon une vue en coupe et de profil, l'appareil de contrôle de la figure 1 dans une première configuration.
[Fig.3] représente, selon la même vue en coupe et de profil que la figure 2, l'appareil de contrôle de la figure 1 dans une deuxième configuration.
[Fig.4] représente, selon une vue en perspective, une partie de l'appareil de contrôle de la figure 1.
[Fig.5] représente, selon une vue en perspective, une variante de réalisation de la partie de l'appareil de contrôle représentée à la figure 4.
[Fig.6] représente, selon une vue de face, un appareil de contrôle selon un deuxième exemple de réalisation de l'invention.
[Fig.7] représente, selon une vue en coupe et de profil selon le plan de coupe AA, l'appareil de contrôle de la figure 6 dans une première configuration.
[Fig.8] représente, selon une vue en coupe et de profil selon le plan de coupe AA, l'appareil de contrôle de la figure 6 dans une deuxième configuration.
[Fig.9] représente, selon une vue en perspective et partielle, un appareil de contrôle de la vision d'un patient selon un troisième exemple de réalisation de l'invention.
[Fig.10] représente l'appareil de contrôle de la figure 9 selon une vue en coupe longitudinale.

### Description détaillée

L'appareil de contrôle 10 permet de tester la vision d'un patient, et notamment de détecter des défauts visuels impactant l'un ou l'autre des yeux du patient et de réalisation un diagnostic substantiel de la vision du patient.

L'appareil de contrôle 10 de la vision d'un patient comprend un boîtier 11 de forme sensiblement parallélépipédique et destiné à abriter et à protéger au moins certains des éléments de l'appareil de contrôle 10.

L'appareil de contrôle 10 comprend des moyens de définition (non représentés) d'une position d'observation d'un œil 50 du patient et d'une position d'observation de l'autre œil 60 du patient. Par exemple, lesdits moyens de définition peuvent comprendre un système de support et de maintien en position de la tête du patient, comme une têtière ou une mentonnière.

L'appareil de contrôle 10 comprend un dispositif d'affichage d'image 70 permettant par exemple d'afficher des optotypes ou autres symboles observables par l'œil 50 et l'autre œil 60 du patient depuis respectivement la position d'observation de l'œil 50 du patient et la position d'observation de l'autre œil 60 du patient.

Le dispositif d'affichage d'image 70 est intégré à l'appareil de contrôle 10, et prévu sur l'une des faces du boîtier 11.

La position d'observation de l'œil 50 du patient et la position d'observation de l'autre œil 60 du patient sont respectivement situées à l'extérieur du boîtier 11.

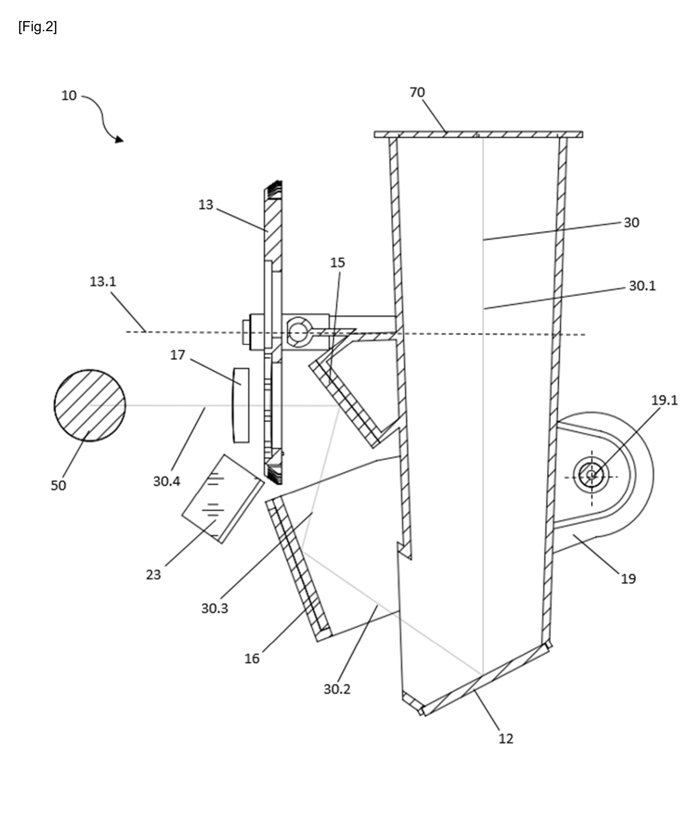

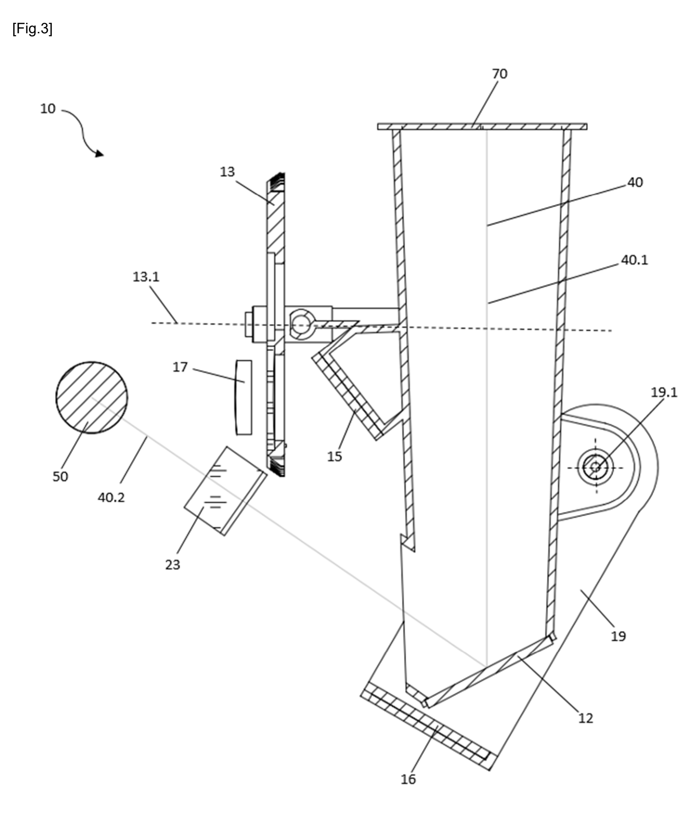

L'appareil de contrôle 10 est prévu pour pouvoir occuper :
- Une première configuration, ou configuration de vision de loin, représentée à la figure 2, dans laquelle un premier chemin optique 30, ou chemin optique de vision de loin, est ménagé entre la position d'observation de l'œil 50 du patient et le dispositif d'affichage d'image 70, et dans laquelle un premier chemin optique supplémentaire (non représenté sur les figures), ou chemin optique de vision de loin supplémentaire, est ménagé entre la position d'observation de l'autre œil 60 du patient et le dispositif d'affichage d'image 70 ;
- Une deuxième configuration, ou configuration de vision de près, représentée à la figure 3, dans laquelle un deuxième chemin optique 40, ou chemin optique de vision de près, est ménagé entre la position d'observation de l'œil 50 du patient et le dispositif d'affichage d'image 70, et dans laquelle un deuxième chemin optique supplémentaire (non représenté sur les figures), ou chemin optique de vision de près supplémentaire, est ménagé entre la position d'observation de l'autre œil 60 du patient et le dispositif d'affichage d'image 70.

Afin de ménager respectivement le deuxième chemin optique 40 et le deuxième chemin optique supplémentaire, le boîtier 11 présente une ouverture de deuxième chemin optique 21 et une ouverture de deuxième chemin optique supplémentaire 22.

Afin de ménager respectivement le premier chemin optique 30 et le premier chemin optique supplémentaire, le boîtier 11 présente également une ouverture de premier chemin optique et une ouverture de premier chemin optique supplémentaire, lesquelles sont non visibles sur les figures.

L'appareil de contrôle 10 comprend un système de renvoi d'image apte à renvoyer les images affichées sur le dispositif d'affichage d'image 70 vers respectivement la position d'observation de l'œil 50 du patient et la position d'observation de l'autre œil 60 du patient.

Plus particulièrement :
- Lorsque l'appareil de contrôle 10 occupe la première configuration, les images renvoyées depuis le dispositif d'affichage d'image 70 empruntent respectivement le premier chemin optique 30 pour atteindre l'œil 50 du patient, et le premier chemin optique supplémentaire pour atteindre l'autre œil 60 du patient ;
- Lorsque l'appareil de contrôle 10 occupe la deuxième configuration, les images renvoyées depuis le dispositif d'affichage d'image 70 empruntent respectivement le deuxième chemin optique 40 pour atteindre l'œil 50 du patient, et le deuxième chemin optique supplémentaire pour atteindre l'autre œil 60 du patient.

Le système de renvoi d'image de l'appareil de contrôle 10 comprend notamment un premier miroir 15 et un deuxième miroir 16.

L'appareil de contrôle 10 comprend également une lentille primaire de premier chemin optique 17 et une lentille primaire de premier chemin optique supplémentaire 18 agencées pour être respectivement disposées sur le premier chemin optique 30 au regard de la position d'observation de l'œil 50 du patient, et sur le premier chemin optique supplémentaire au regard de la position d'observation de l'autre œil 60 du patient. L'appareil de contrôle 10 comprend en outre un organe mobile 13 présentant cinq logements d'organe mobile 13.2 destinés à occuper alternativement sur le premier chemin optique 30 une position de logement par rotation de l'organe mobile 13 autour d'un axe de rotation d'organe mobile 13.1 sensiblement coplanaire et parallèle à une direction d'une portion du premier chemin optique s'étendant entre la position d'observation de l'œil 50 du patient et la position de logement.

Les cinq logements d'organe mobile 13.2 sont identiques entre eux, chacun étant respectivement apte à accueillir un élément optique, comme par exemple une lentille.

L'appareil de contrôle 10 comprend également un organe mobile supplémentaire 14 présentant cinq logements d'organe mobile supplémentaire 14.2 destinés à occuper alternativement sur le premier chemin optique supplémentaire une position de logement supplémentaire par rotation de l'organe mobile supplémentaire 14 autour d'un axe de rotation d'organe mobile supplémentaire 14.1 sensiblement coplanaire avec et parallèle à une direction d'une portion du premier chemin optique supplémentaire s'étendant entre la position d'observation de l'autre œil 60 du patient et la position de logement supplémentaire.

La lentille primaire de premier chemin optique 17 est disposée sur le premier chemin optique 30 entre la position d'observation de l'œil 50 du patient et la position de logement, et la lentille primaire de premier chemin optique supplémentaire 18 est disposée sur le premier chemin optique supplémentaire entre la position d'observation de l'autre œil 60 du patient et la position de logement supplémentaire.

Les cinq logements d'organe mobile supplémentaires 14.2 sont identiques entre eux, et également identiques aux cinq logements d'organe mobile 13.2, chacun des cinq logements d'organe mobile supplémentaires 14.2 étant respectivement apte à accueillir un élément optique, comme par exemple une lentille.

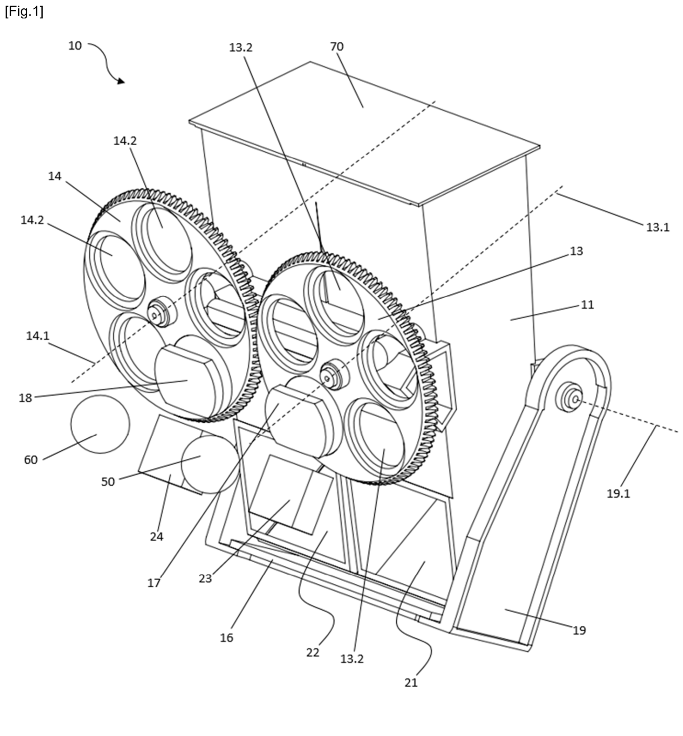

Les cinq logements d'organe mobile 13.2 sont représentés à la figure 1 dépourvus d'éléments optiques.

Les cinq logements d'organe mobile supplémentaire 14.2 sont représentés à la figure 1 dépourvus d'éléments optiques.

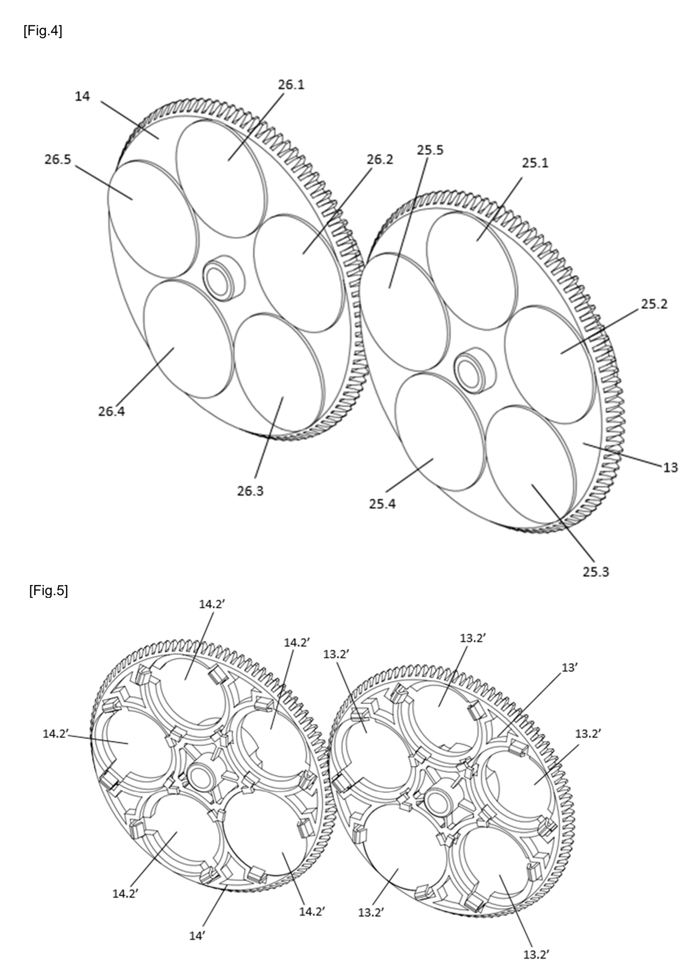

L'organe mobile 13 et l'organe mobile supplémentaire 14, qui sont donc respectivement représentés à la figure 1 totalement dépourvus d'éléments optiques, sont également représentés à la figure 4 respectivement pourvus de cinq lentilles secondaires de premier chemin optique 25.1, 25.2, 25.3, 25.4, 25.5 et cinq lentilles secondaires de premier chemin optique 26.1, 26.2, 26.3, 26.4, 26.5.

Les cinq lentilles secondaires de premier chemin optique 25.1, 25.2, 25.3, 25.4, 25.5 sont respectivement collées dans chacun des logements d'organe mobile 13.2, tandis que les cinq lentilles secondaires de premier chemin optique supplémentaire 26.1, 26.2, 26.3, 26.4, 26.5 sont respectivement collées dans chacun des logements d'organe mobile supplémentaires 14.2.

L'organe mobile 13' et l'organe mobile supplémentaire 14' représentés à la figure 5 sont respectivement une variante de réalisation de l'organe mobile 13 et de l'organe mobile supplémentaire 14, et présentent respectivement des logements d'organe mobile 13.2' et des logements d'organe mobile supplémentaire 14.2' permettant d'accueillir temporairement des lentilles secondaires de premier chemin optique et des lentilles secondaires de premier chemin optique supplémentaire, respectivement.

En effet, chacun des logements d'organe mobile 13.2' et des logements d'organe mobile supplémentaire 14.2' sont identiques entre eux et présente un système de fixation temporaire pour lentille permettant de clipper des lentilles.

L'organe mobile 13, 13' et l'organe mobile supplémentaire 14, 14' sont configurés entre eux pour être mobiles en rotation de manière synchronisée. En d'autres termes, un mouvement en rotation de l'organe mobile 13, 13' (respectivement de l'organe mobile supplémentaire 14, 14') entraine en rotation, de manière synchronisée, l'organe mobile supplémentaire 14, 14' (respectivement l'organe mobile 13, 13').

Ainsi, lorsque l'organe mobile 13, 13' se déplace en rotation à une vitesse donnée, l'organe mobile 14, 14' se déplace également en rotation à la même vitesse, et en sens inverse.

La mobilité en rotation synchronisée de l'organe mobile 13, 13' et de l'organe mobile supplémentaire 14, 14' est obtenue grâce à un mécanisme d'engrenage, une première partie de mécanisme d'engrenage étant prévue sur l'organe mobile 13, 13', et une deuxième partie de mécanisme d'engrenage, complémentaire de la première partie de mécanisme d'engrenage, étant prévue sur l'organe mobile supplémentaire 14, 14'.

Précisément, la première partie de mécanisme d'engrenage comprend un premier système de dents disposé à la périphérie, et en particulier sur la circonférence, de l'organe mobile 13, 13', et la deuxième partie de mécanisme d'engrenage comprend un deuxième système de dents complémentaire dudit premier système de dents et disposé à la périphérie, et en particulier sur la circonférence, de l'organe mobile supplémentaire 14, 14'.

L'appareil de contrôle 10 comprend en outre des moyens d'actionnement et des moyens d'entrainement en rotation de l'organe mobile 13, 13' et/ou de l'organe mobile supplémentaire 14, 14'.

Le système de renvoi d'image de l'appareil de contrôle 10 est partiellement mobile de manière à faire passer l'appareil de contrôle 10 de la première configuration à la deuxième configuration, et vice versa.

Le premier miroir 15 du système de renvoi d'image est fixe, tandis que le deuxième miroir 16 du système de renvoi d'image est mobile.

En effet, alors que le premier miroir 15 est monté sur un support fixe, le deuxième miroir 16 est monté sur un support de deuxième miroir 19 mobile en rotation autour d'un axe de rotation de support de deuxième miroir 19.1.

L'appareil de contrôle 10 comprend un dispositif d'actionnement de support de deuxième miroir et un dispositif d'entrainement de support de deuxième miroir (non représentés) comprenant au moins un moteur.

En particulier, le deuxième miroir 16 est mobile entre une position déployée de deuxième miroir (visible sur la figure 2) occupée par le deuxième miroir 16 lorsque l'appareil de contrôle 10 occupe la première configuration, et une position escamotée de deuxième miroir (visible sur la figure 3) occupée par le deuxième miroir 16 lorsque l'appareil de contrôle 10 occupe la deuxième configuration.

Le support de deuxième miroir 19 est apte à faire passer le deuxième miroir 16 de la position déployée de deuxième miroir à la position escamotée de deuxième miroir, et inversement.

Le système de renvoi d'image comprend en outre un troisième miroir 12 disposé sur le premier chemin optique 30 entre le dispositif d'affichage d'image 70 et le deuxième miroir 16 lorsque le deuxième miroir 16 occupe la position déployée de deuxième miroir, c'est-à-dire lorsque l'appareil de contrôle 10 occupe la première configuration.

En outre, lorsque le deuxième miroir 16 occupe la position escamotée de deuxième miroir, c'est-à-dire lorsque l'appareil de contrôle 10 occupe la deuxième configuration, le troisième miroir 12 est disposé sur le deuxième chemin optique 40.

L'appareil de contrôle 10 comprend aussi un prisme de deuxième chemin optique 23 monté de manière amovible (et donc interchangeable) sur un support de prisme de deuxième chemin optique (non référencé) et disposé sur le deuxième chemin optique 40 entre le troisième miroir 12 et la position d'observation de l'œil 50 du patient, à proximité de l'ouverture de deuxième chemin optique 21.

De manière similaire, l'appareil de contrôle 10 comprend aussi un prisme de deuxième chemin optique supplémentaire 24 monté de manière amovible (et donc interchangeable) sur un support de prisme de deuxième chemin optique supplémentaire (non référencé) et disposé sur le deuxième chemin optique supplémentaire entre le troisième miroir 12 et la position d'observation de l'autre œil 60 du patient, à proximité de l'ouverture de deuxième chemin optique supplémentaire 22.

Ainsi, lorsque le deuxième miroir 16 occupe la position escamotée de deuxième miroir, c'est-à-dire lorsque l'appareil de contrôle 10 occupe la deuxième configuration, le troisième miroir 12 est disposé sur le deuxième chemin optique 40 entre le dispositif d'affichage d'image 70 et le prisme de deuxième chemin optique 23 ainsi que sur le deuxième chemin optique supplémentaire entre le dispositif d'affichage d'image 70 et le prisme de deuxième chemin optique supplémentaire 24.

Le système de renvoi d'image est apte à renvoyer l'image ou les images affichées sur le dispositif d'affichage d'image 70 grâce en partie notamment à l'agencement particulier des premier, deuxième et troisième miroirs (15, 16, 12).

Lorsque l'appareil de contrôle 10 occupe la première configuration, le troisième miroir 12 est prévu pour coopérer avec le deuxième miroir 16 occupant la position déployée de deuxième miroir, et le deuxième miroir 16 occupant la position déployée de deuxième miroir est prévu pour coopérer avec le premier miroir 15. En outre, le deuxième miroir 16 occupant la position déployée de deuxième miroir obstrue le deuxième chemin optique 40 ainsi que le deuxième chemin optique supplémentaire.

Comme cela est visible sur la figure 2, le premier chemin optique 30 comprend donc :
- Une première portion de premier chemin optique 30.1 s'étendant entre le dispositif d'affichage d'image 70 et le troisième miroir 12 ;
- Une deuxième portion de premier chemin optique 30.2 s'étendant entre le troisième miroir 12 et le deuxième miroir 16 ;
- Une troisième portion de premier chemin optique 30.3 s'étendant entre le deuxième miroir 16 et le premier miroir 15 ;
- Une quatrième portion de premier chemin optique 30.4 s'étendant entre le premier miroir 15 et la position d'observation de l'œil 50 du patient, en passant par la position de logement et la lentille primaire de premier chemin optique 17.

Le premier chemin optique supplémentaire s'étend en outre parallèlement au premier chemin optique 30, une quatrième portion de premier chemin optique supplémentaire s'étendant entre le premier miroir 15 et la position d'observation de l'autre œil 60 du patient, en passant par la position de logement supplémentaire et la lentille primaire de premier chemin optique supplémentaire 18.

Lorsque l'appareil de contrôle 10 occupe la deuxième configuration, le deuxième miroir 16 occupe la position escamotée de deuxième miroir, et le troisième miroir est prévu pour renvoyer les images affichées sur le dispositif d'affichage d'image directement vers la position d'observation de l'œil 50 du patient. De plus, lorsqu'il occupe la position escamotée de deuxième miroir, le deuxième miroir 16 libère le deuxième chemin optique 40 ainsi que le deuxième chemin optique supplémentaire, et tant le premier chemin optique 30 que le premier chemin optique supplémentaire sont interrompus.

Ainsi, et comme cela est visible sur la figure 3, le deuxième chemin optique 40 comprend :
- Une première portion de deuxième chemin optique 40.1 s'étendant entre le dispositif d'affichage d'image 70 et le troisième miroir 12 ;
- Une deuxième portion de deuxième chemin optique 40.2 s'étendant entre le troisième miroir 12 et la position d'observation de l'œil 50 du patient, en passant par le prisme de deuxième chemin optique 23.

Le deuxième chemin optique supplémentaire s'étend en outre parallèlement au deuxième chemin optique 40, une deuxième portion de deuxième chemin optique supplémentaire s'étendant entre le troisième miroir 12 et la position d'observation de l'autre œil 60 du patient, en passant par le prisme de deuxième chemin optique supplémentaire 24.

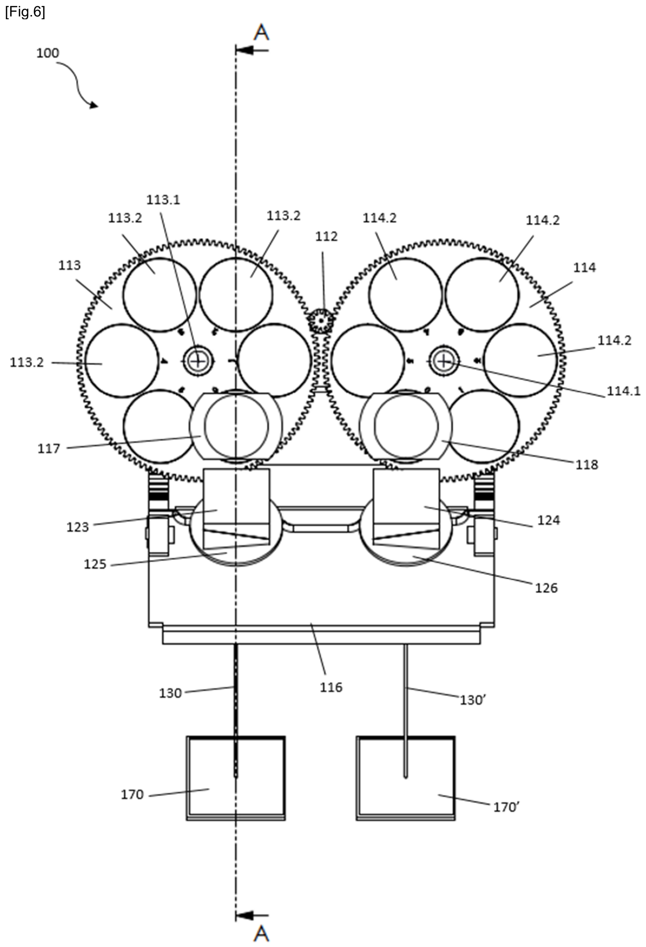

Comme l'appareil de contrôle 10, l'appareil de contrôle 100 des figures 6 à 8 permet de tester de manière globale la vision d'un patient, et notamment de détecter des défauts visuels impactant l'un ou l'autre des yeux du patient et/ou de réaliser un diagnostic complet de la vision du patient.

Certains éléments communs à l'appareil de contrôle 10 et à l'appareil de contrôle 100, déjà décrits ci-avant, ne seront pas de nouveau décrits en détails ci-après.

L'appareil de contrôle 100 comprend des moyens de définition (non représentés) d'une position d'observation d'un œil 150 du patient et d'une position d'observation de l'autre œil (non représenté) du patient.

Un dispositif d'affichage d'image 170, déporté de l'appareil de contrôle 100, permet par exemple d'afficher des optotypes ou autres symboles observables par l'œil 150 du patient depuis la position d'observation de l'œil 150 du patient. De plus, un dispositif d'affichage d'image supplémentaire 170' permet par exemple d'afficher des optotypes ou autres symboles observables par l'autre oeil du patient depuis la position d'observation de l'autre œil du patient.

L'appareil de contrôle 100 comprend un boîtier (non représenté) destiné à abriter et à protéger au moins certains des éléments de l'appareil de contrôle 100, et tant la position d'observation de l'œil 150 du patient que la position d'observation de l'autre œil du patient sont situées à l'extérieur de ce boîtier.

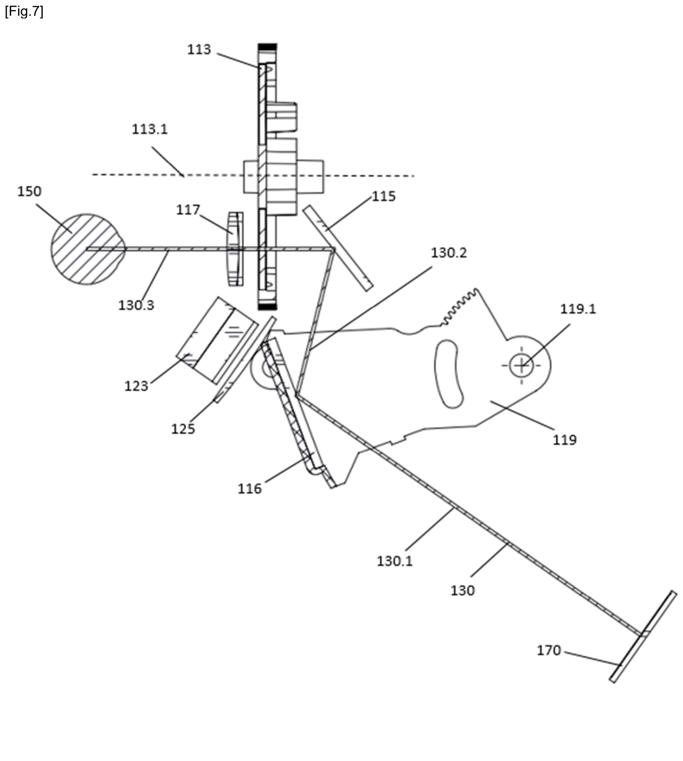

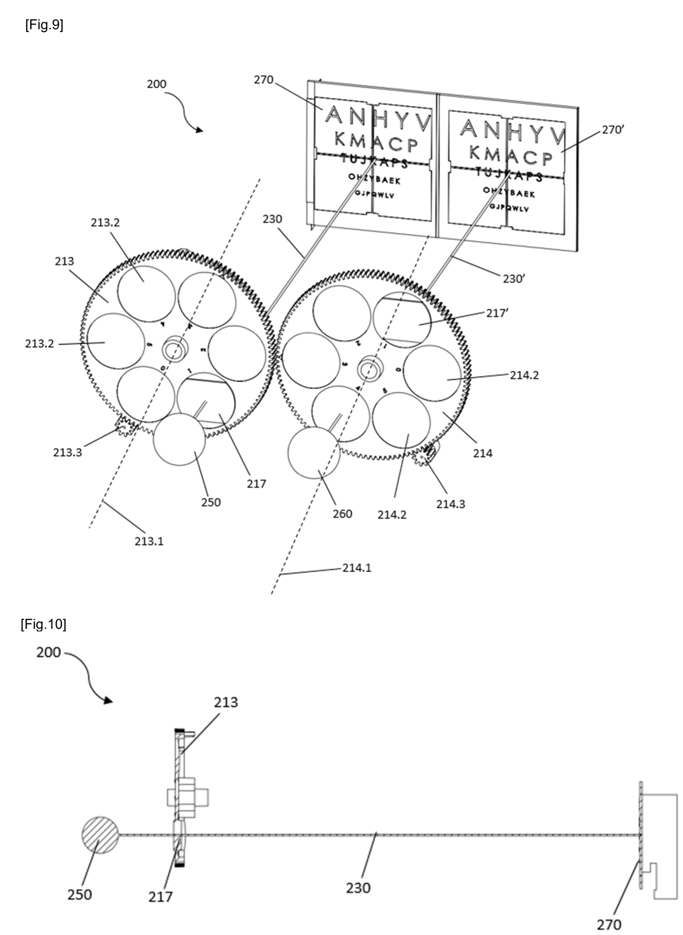

L'appareil de contrôle 100 est prévu pour pouvoir occuper :
- Une première configuration, ou configuration de vision de loin, représentée à la figure 7, dans laquelle un premier chemin optique 130, ou chemin optique de vision de loin, est ménagé entre la position d'observation de l'œil 150 du patient et le dispositif d'affichage d'image 170, et dans laquelle un premier chemin optique supplémentaire 130', ou chemin optique de vision de loin supplémentaire, est ménagé entre la position d'observation de l'autre œil du patient et le dispositif d'affichage d'image supplémentaire 170' ;
- Une deuxième configuration, ou configuration de vision de près, représentée à la figure 10, dans laquelle un deuxième chemin optique 140, ou chemin optique de vision de près, est ménagé entre la position d'observation de l'œil 150 du patient et le dispositif d'affichage d'image 170, et dans laquelle un deuxième chemin optique supplémentaire, ou chemin optique de vision de près supplémentaire, (non représenté sur les figures) est ménagé entre la position d'observation de l'autre œil du patient et le dispositif d'affichage d'image supplémentaire 170'.

Afin de ménager respectivement le premier chemin optique 130, le premier chemin optique supplémentaire 130', le deuxième chemin optique 140 et le deuxième chemin optique supplémentaire, le boîtier de l'appareil de contrôle 100 présente différentes ouvertures de premier chemin optique et différentes ouvertures de deuxième chemin optique, non représentées sur les figures. L'appareil de contrôle 100 comprend aussi un système de renvoi d'image apte d'une part à renvoyer les images affichées sur le dispositif d'affichage d'image 170 vers respectivement la position d'observation de l'œil 150 du patient et la position d'observation de l'autre œil du patient, et apte d'autre part à renvoyer les images affichées sur le dispositif d'affichage d'image supplémentaire 170' vers respectivement la position d'observation de l'œil 150 du patient et la position d'observation de l'autre œil du patient.

Plus particulièrement :
- Lorsque l'appareil de contrôle 100 occupe la première configuration, les images renvoyées depuis le dispositif d'affichage d'image 170 empruntent le premier chemin optique 130 pour atteindre l'œil 150 du patient, et les images renvoyées depuis le dispositif d'affichage d'image supplémentaire 170' empruntent le premier chemin optique supplémentaire 130' pour atteindre l'autre œil du patient ;
- Lorsque l'appareil de contrôle 100 occupe la deuxième configuration, les images renvoyées depuis le dispositif d'affichage d'image 170 empruntent le deuxième chemin optique 140 pour atteindre l'œil 150 du patient, et les images renvoyées depuis le dispositif d'affichage d'image supplémentaire 170' empruntent le deuxième chemin optique supplémentaire pour atteindre l'autre œil du patient.

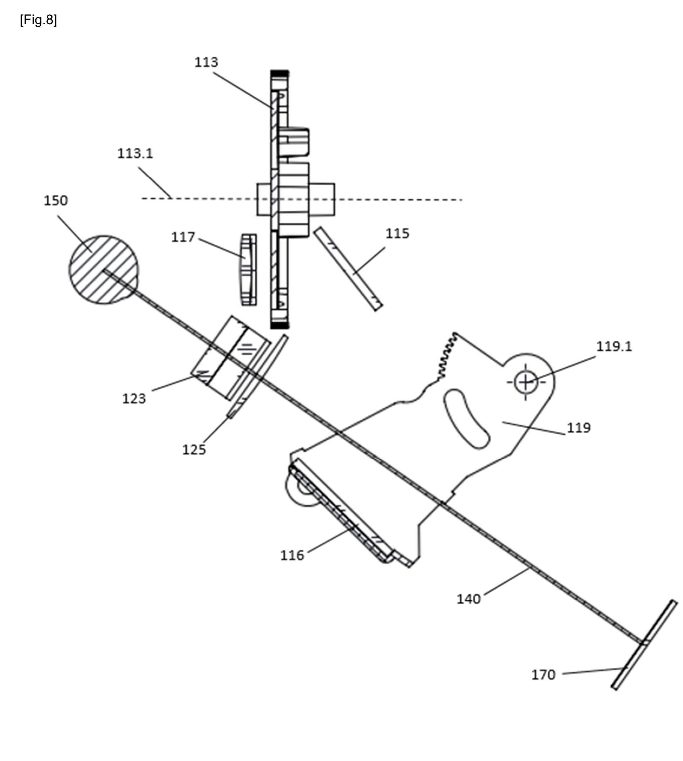

Le système de renvoi d'image comprend un premier miroir 115 (visible sur les figures 7 et 8) et un deuxième miroir 116.

L'appareil de contrôle 100 comprend également une lentille primaire de premier chemin optique 117 et une lentille primaire de premier chemin optique supplémentaire 118 agencées pour être respectivement disposées sur le premier chemin optique 130 au regard de la position d'observation de l'œil 150 du patient, et sur le premier chemin optique supplémentaire 130' au regard de la position d'observation de l'autre œil du patient.

L'appareil de contrôle 100 comprend en outre un organe mobile 113 présentant six logements d'organe mobile 113.2 destinés à occuper alternativement sur le premier chemin optique 130 une position de logement par rotation de l'organe mobile 113 autour d'un axe de rotation d'organe mobile 113.1 sensiblement coplanaire avec et parallèle à une direction d'une portion du premier chemin optique s'étendant entre la position d'observation de l'œil 150 du patient et la position de logement.

Les six logements d'organe mobile 113.2 sont identiques entre eux, chacun étant respectivement apte à accueillir un élément optique (non représenté), comme par exemple une lentille.

L'appareil de contrôle 100 comprend également un organe mobile supplémentaire 114 présentant six logements d'organe mobile supplémentaire 114.2 destinés à occuper alternativement sur le premier chemin optique supplémentaire 130' une position de logement supplémentaire par rotation de l'organe mobile supplémentaire 114 autour d'un axe de rotation d'organe mobile supplémentaire 114.1 sensiblement coplanaire avec et parallèle à une direction d'une portion du premier chemin optique supplémentaire (non visible sur les figures) s'étendant entre la position d'observation de l'autre œil du patient et la position de logement supplémentaire.

La lentille primaire de premier chemin optique 117 est disposée sur le premier chemin optique 130 entre la position d'observation de l'œil 150 du patient et la position de logement, et la lentille primaire de premier chemin optique supplémentaire 118 est disposée sur le premier chemin optique supplémentaire 130' entre la position d'observation de l'autre œil du patient et la position de logement supplémentaire.

Les six logements d'organe mobile supplémentaires 114.2 sont identiques entre eux, et également identiques aux six logements d'organe mobile 113.2, chacun des six logements d'organe mobile supplémentaires 114.2 étant respectivement apte à accueillir un élément optique, comme par exemple une lentille.

Les six logements d'organe mobile 113.2 sont représentés à la figure 6 dépourvus d'éléments optiques.

Les six logements d'organe mobile 114.2 sont représentés à la figure 6 dépourvus d'éléments optiques.

Le système de renvoi d'image est partiellement mobile de manière à faire passer l'appareil de contrôle 100 de la première configuration à la deuxième configuration, et vice versa.

Le premier miroir 115 du système de renvoi d'image est fixe, tandis que le deuxième miroir 116 du système de renvoi d'image est mobile.

En effet, alors que le premier miroir 115 est monté sur un support de premier miroir fixe, le deuxième miroir 116 est monté sur un support de deuxième miroir 119 mobile en rotation autour d'un axe de rotation de support de deuxième miroir 119.1.

L'appareil de contrôle 100 comprend un dispositif d'actionnement de support de deuxième miroir et un dispositif d'entrainement de support de deuxième miroir (non représentés) comprenant au moins un moteur.

En particulier, le deuxième miroir 116 est mobile entre une position déployée de deuxième miroir (visible sur la figure 7) occupée par le deuxième miroir 116 lorsque l'appareil de contrôle 100 occupe la première configuration, et une position escamotée de deuxième miroir (visible sur la figure 8) occupée par le deuxième miroir 116 lorsque l'appareil de contrôle 100 occupe la deuxième configuration.

Le support de deuxième miroir 119 est donc apte à faire passer le deuxième miroir 116 de la position déployée de deuxième miroir à la position escamotée de deuxième miroir, et inversement.

L'appareil de contrôle 100 comprend aussi un prisme de deuxième chemin optique 123 monté de manière amovible (et donc interchangeable) sur un support de prisme de deuxième chemin optique (non référencé) et disposé sur le deuxième chemin optique 140. L'appareil de contrôle 100 comprend également une lentille de deuxième chemin optique 125 montée de manière amovible sur un support de lentille de deuxième chemin optique (non représenté), disposée sur le deuxième chemin optique 140 et associée au prisme de deuxième chemin optique 123

De manière similaire, l'appareil de contrôle 100 comprend aussi un prisme de deuxième chemin optique supplémentaire 124 monté de manière amovible (et donc interchangeable) sur un support de prisme de deuxième chemin optique supplémentaire (non référencé) et disposé sur le deuxième chemin optique supplémentaire. L'appareil de contrôle 100 comprend également une lentille de deuxième chemin optique supplémentaire 126 montée de manière amovible sur un support de lentille de deuxième chemin optique supplémentaire (non représenté), disposée sur le deuxième chemin optique supplémentaire et associée au prisme de deuxième chemin optique supplémentaire 124.

Lorsque l'appareil de contrôle 100 occupe la première configuration, le deuxième miroir 116 occupant la position déployée de deuxième miroir est prévu pour coopérer avec le premier miroir 115. En outre, le deuxième miroir 116 occupant la position déployée de deuxième miroir obstrue le deuxième chemin optique 140 ainsi que le deuxième chemin optique supplémentaire.

Comme cela est visible sur la figure 7, le premier chemin optique 130 comprend donc :
- Une première portion de premier chemin optique 130.1 s'étendant entre le dispositif d'affichage d'image 170 et le deuxième miroir 116 ;
- Une deuxième portion de premier chemin optique 130.2 s'étendant entre le deuxième miroir 116 et le premier miroir 115 ;
- Une troisième portion de premier chemin optique 130.3 s'étendant entre le premier miroir 15 et la position d'observation de l'œil 150 du patient, en passant par la position de logement et la lentille primaire de premier chemin optique 117.

Le premier chemin optique supplémentaire 130' s'étend en outre parallèlement au premier chemin optique 130 en partant du dispositif d'affichage supplémentaire 170', une troisième portion de premier chemin optique supplémentaire s'étendant entre le premier miroir 115 et la position d'observation de l'autre œil du patient, en passant par la position de logement supplémentaire et la lentille primaire de premier chemin optique supplémentaire 118.

Lorsque l'appareil de contrôle 100 occupe la deuxième configuration, le deuxième miroir 116 occupe la position escamotée de deuxième miroir, les images affichées sur le dispositif d'affichage d'image 170 étant directement renvoyées vers la position d'observation de l'œil 150 du patient en passant à travers la lentille de deuxième chemin optique 125 et le prisme de deuxième chemin optique 123. De plus, lorsqu'il occupe la position escamotée de deuxième miroir, le deuxième miroir 116 libère le deuxième chemin optique 140 ainsi que le deuxième chemin optique supplémentaire, et tant le premier chemin optique 130 que le premier chemin optique supplémentaire 130' sont interrompus.

Ainsi, et comme cela est visible sur la figure 8, le deuxième chemin optique 140 s'étend directement entre le dispositif d'affichage d'image 170 et la position d'observation de l'œil 150 du patient, en passant à travers la lentille de deuxième chemin optique 125 et le prisme de deuxième chemin optique 123.

Le deuxième chemin optique supplémentaire s'étend en outre parallèlement au deuxième chemin optique 140, directement entre le dispositif d'affichage d'image supplémentaire 170' et la position d'observation de l'autre œil du patient, en passant à travers la lentille de deuxième chemin optique supplémentaire 126 et le prisme de deuxième chemin optique supplémentaire 124.

L'organe mobile 113 et l'organe mobile supplémentaire 114 sont configurés entre eux pour être mobiles en rotation de manière synchronisée. En d'autres termes, un mouvement en rotation de l'organe mobile 113 (respectivement de l'organe mobile supplémentaire 114) entraine en rotation, de manière synchronisée, l'organe mobile supplémentaire 114 (respectivement l'organe mobile 113).

Ainsi, lorsque l'organe mobile 113 se déplace en rotation à une vitesse donnée, l'organe mobile 114 se déplace également en rotation à la même vitesse.

La mobilité en rotation synchronisée de l'organe mobile 113 et de l'organe mobile supplémentaire 114 est obtenu grâce respectivement à un mécanisme d'entrainement, une première partie de mécanisme d'entrainement étant prévue sur l'organe mobile 113, une deuxième partie de mécanisme d'entrainement prévue sur l'organe mobile supplémentaire 114, et une troisième partie de mécanisme d'entrainement comprenant un pignon 112 mobile en rotation complémentaire à la fois de la première partie de mécanisme d'entrainement et de la deuxième partie de mécanisme d'entrainement.

Précisément, la première partie de mécanisme d'entrainement comprend un premier système de dents disposé à la périphérie, et en particulier sur la circonférence, de l'organe mobile 113, la deuxième partie de mécanisme d'entrainement comprend un deuxième système de dents disposé à la périphérie, et en particulier sur la circonférence, de l'organe mobile supplémentaire 114, et le pignon 112 comprend un troisième système de dents complémentaire à la fois du premier système de dents et du deuxième système de dents.

Précisément, le pignon 112 présente une forme de disque de faible épaisseur et comprend à sa périphérie, en particulier sur sa circonférence, le troisième système de dents.

Grâce à cet agencement et en particulier au pignon 112 mobile en rotation, le sens de rotation de l'organe mobile supplémentaire 114 est identique au sens de rotation de l'organe mobile 113. De plus, l'organe mobile supplémentaire 114 est strictement identique à l'organe mobile 113.

Enfin, l'appareil de contrôle 100 comprend un dispositif d'actionnement de pignon et un dispositif d'entrainement de pignon (non représenté) comprenant au moins un moteur. Ainsi, le pignon 112 est motorisé.

L'appareil de contrôle 200 de la vision d'un patient, visible sur les figures 9 et 10 est une version plus simple et moins coûteuse que les appareils de contrôle 10 et 100 précédemment décrit, et pourra ainsi être avantageusement utilisé à des fins d'opérations de dépistage à grande échelle, impliquant par exemple une grande partie d'une population donnée.

L'appareil de contrôle 200 est notamment dépourvu d'élément optique primaire de premier chemin optique.

Certains éléments communs aux appareils de contrôle 10, 100, 200, déjà décrits ci-avant, ne seront pas de nouveau décrits en détails ci-après.

L'appareil de contrôle 200 est prévu pour pouvoir occuper une première configuration dans laquelle un premier chemin optique 230, pouvant être un chemin optique de vision de près, de vision de loin ou de vision intermédiaire, est ménagé entre une position d'observation d'un œil 250 du patient et un dispositif d'affichage d'image 270 déporté.

L'appareil de contrôle 200 comprend un organe mobile 213 présentant six logements d'organe mobile 213.2 destinés à occuper alternativement sur le premier chemin optique 230 une position de logement par rotation de l'organe mobile 213 autour d'un axe de rotation d'organe mobile 213.1 sensiblement coplanaire et parallèle à une direction d'une portion du premier chemin optique 230 s'étendant entre la position d'observation de l'œil 250 du patient et la position de logement.

Chacun des six logements d'organe mobile 213.2 intègre en permanence un élément optique secondaire de premier chemin optique, par exemple une lentille secondaire de premier chemin optique ou un prisme de premier chemin optique 217.

De plus, un premier chemin optique supplémentaire 230', pouvant être un chemin optique de vision de près, de vision de loin ou de vision intermédiaire, est également ménagé entre une position d'observation de l'autre œil 260 du patient et un dispositif d'affichage d'image supplémentaire 270' déporté, et l'appareil de contrôle comprend aussi un organe mobile supplémentaire 214 présentant six logements d'organe mobile supplémentaire 214.2 destinés à occuper alternativement sur le premier chemin optique supplémentaire 230' une position de logement supplémentaire par rotation de l'organe mobile supplémentaire 214 autour d'un axe de rotation d'organe mobile supplémentaire 214.1 sensiblement coplanaire et parallèle à une direction d'une portion du premier chemin optique supplémentaire 230' s'étendant entre la position d'observation de l'autre œil 260 du patient et la position de logement supplémentaire.

Chacun des six logements d'organe mobile supplémentaire 214.2 intègre en permanence un élément optique secondaire de premier chemin optique supplémentaire, par exemple une lentille secondaire de premier chemin optique supplémentaire ou un prisme de premier chemin optique supplémentaire 217'.

On comprend qu'en fonction de la nature et/ou des propriétés des éléments optiques secondaires de premier chemin optique prévus sur l'appareil de contrôle 200, ce dernier puisse permettre de tester, en occupant une seule et même configuration, la vision de près, la vision de loin ou encore la vision intermédiaire, et ce par simple rotation de l'organe mobile 213 et/ou de l'organe mobile supplémentaire 214.

L'organe mobile 213 et l'organe mobile supplémentaire 214 de l'appareil de contrôle sont configurés pour être mobiles en rotation indépendamment l'un de l'autre. En d'autres termes, les mouvements respectifs de l'organe mobile 213 et de l'organe mobile supplémentaire sont désynchronisés, et l'organe mobile 213 peut se déplacer alors que l'organe mobile supplémentaire reste fixe, ou inversement.

De plus, les vitesses de rotation respectives de l'organe mobile et de l'organe mobile supplémentaire ne sont pas forcément identiques.

L'organe mobile 213 dispose de son propre mécanisme d'entrainement, en particulier composé d'un pignon 213.3 motorisé ou non.

De manière similaire, l'organe mobile supplémentaire 214 dispose également de son propre mécanisme d'entrainement composé d'un pignon 214.3 motorisé ou non.

L'invention ne se limite évidemment pas aux trois modes de réalisation représentés sur les figures et décrits ci-dessus, et englobe toutes les variantes de réalisation envisageables par l'homme du métier. En particulier, les différents éléments non incompatibles décrits ci-dessus pourront être combinés dans autant de variantes de réalisation.

## Revendications

1. Appareil de contrôle (10, 100) de la vision d'un patient prévu pour pouvoir occuper au moins une première configuration dans laquelle un premier chemin optique (30, 130) est ménagé entre une position d'observation d'un œil (50, 150) du patient et un dispositif d'affichage d'image (70, 170), l'appareil de contrôle (10, 100) comprenant un organe mobile (13, 13', 113) présentant au moins deux logements d'organe mobile (13.2, 13.2', 113.2) destinés à occuper alternativement sur le premier chemin optique (30, 130) une position de logement par rotation de l'organe mobile (13, 13', 113) autour d'un axe de rotation d'organe mobile (13.1, 113.1) sensiblement coplanaire et parallèle à une direction d'une portion du premier chemin optique (30, 130) s'étendant entre la position d'observation de l'œil (50, 150) du patient et la position de logement, l'appareil de contrôle (10, 100) étant prévu pour pouvoir occuper en outre une deuxième configuration dans laquelle un deuxième chemin optique (40, 140) est ménagé entre la position d'observation de l'œil (50, 150) du patient et le dispositif d'affichage d'image (70, 170), l'appareil de contrôle (10, 100) comprenant en outre un système de renvoi d'image comprenant un premier miroir (15, 115) et un deuxième miroir (16, 116), le deuxième miroir (16, 116) étant mobile entre :
- Une position déployée de deuxième miroir occupée par le deuxième miroir (16, 116) lorsque l'appareil de contrôle (10, 100) occupe la première configuration, et
- Une position escamotée de deuxième miroir occupée par le deuxième miroir (16, 116) lorsque l'appareil de contrôle (10, 100) occupe la deuxième configuration

2. Appareil de contrôle (10, 100) selon la revendication 1 comprenant en outre un élément optique primaire de premier chemin optique disposé sur le premier chemin optique.

3. Appareil de contrôle (10, 100) selon la revendication 1 ou la revendication 2, dans lequel :
- Chacun desdits au moins deux logements d'organe mobile (13.2, 113.2) intègre en permanence un élément optique secondaire de premier chemin optique, ou dans lequel :
- Chacun desdits au moins deux logements d'organe mobile (13.2') est apte à accueillir temporairement un élément optique secondaire de premier chemin optique.

4. Appareil de contrôle (10, 100) selon la revendication 2, dans lequel l'élément optique primaire de premier chemin optique est une lentille primaire de premier chemin optique (17, 117).

5. Appareil de contrôle (10, 100) selon la revendication 3, dans lequel l'élément optique secondaire de premier chemin optique est une lentille secondaire de premier chemin optique (25.1, 25.2, 25.3, 25.4, 25.5) ou un prisme de premier chemin optique (217).

6. Appareil de contrôle (10, 100) selon l'une des revendications précédentes, comprenant au moins un élément optique de deuxième chemin optique disposé sur le deuxième chemin optique (40, 140).

7. Appareil de contrôle (10, 100) selon la revendication 6, dans lequel l'au moins un élément optique de deuxième chemin optique comprend un prisme de deuxième chemin optique (23, 123) et/ou une lentille de deuxième chemin optique (125).

8. Appareil de contrôle (10, 100) selon l'une des revendications précédentes, dans lequel le système de renvoi d'image comprend un troisième miroir (12).

9. Appareil de contrôle (10, 100) selon l'une des revendications précédentes, dans lequel, lorsque l'appareil de contrôle (10, 100) occupe la première configuration, un premier chemin optique supplémentaire (30', 130') est ménagé entre une position d'observation de l'autre œil (60) du patient et le dispositif d'affichage d'image (70), l'appareil de contrôle (10, 100) comprenant un organe mobile supplémentaire (14, 14', 114) comprenant au moins deux logements d'organe mobile supplémentaire (14.2, 14.2', 114.2) destinés à occuper alternativement sur le premier chemin optique supplémentaire (30', 130') une position de logement supplémentaire, par rotation de l'organe mobile supplémentaire (14, 14', 114) autour d'un axe de rotation d'organe mobile supplémentaire (14.1, 114.1) sensiblement coplanaire et parallèle à une direction d'une portion du premier chemin optique supplémentaire s'étendant entre la position d'observation de l'autre œil (60) du patient et la position de logement supplémentaire.

10. Appareil de contrôle (10, 100) selon la revendication 9, dans lequel l'organe mobile (13, 13', 113) et l'organe mobile supplémentaire (14, 14', 114) sont configurés entre eux pour être mobiles en rotation de manière synchronisée.

11. Appareil de contrôle (10, 100) selon l'une des revendications 9 ou 10, comprenant un mécanisme d'entrainement apte à entrainer simultanément en rotation l'organe mobile (13, 13', 113) et l'organe mobile supplémentaire (14, 14', 114).

12. Appareil de contrôle (10, 100) selon la revendication 11, dans lequel le mécanisme d'entrainement comprend une première partie de mécanisme d'entrainement prévue sur l'organe mobile (113), une deuxième partie de mécanisme d'entrainement prévue sur l'organe mobile supplémentaire (114), et une troisième partie de mécanisme d'entrainement comprenant un pignon (112) mobile en rotation.

13. Procédé d'utilisation d'un appareil de contrôle conforme à l'une des revendications précédentes.
